# EUROPÄISCHE PATENTANMELDUNG

(11) **EP 0 564 439 A2**
(43) Veröffentlichungstag der Anmeldung: **06.10.1993**
(21) Anmeldenummer: 93890059.4
(22) Anmeldetag: 29.03.1993
(51) Int. Cl.: G01N 1/00, G01N 33/487

(54) **Einrichtung zur wahlweisen Beschickung eines Analysengerätes mit Flüssigkeiten**

(30) Priorität: 02.04.1992 AT 683/92
(71) Anmelder: AVL Medical Instruments AG, CH-8207 Schaffhausen (CH)
(72) Erfinder: Marsoner, Hermann, Dipl.-Ing. Dr., A-8151 Steinberg (AT); Kleinhappl, Erich, A-8062 Weinitzen (AT); Löschnigg, Rainhard, Dipl.-Ing., A-8051 Graz (AT)
(74) Vertreter: Krause, Walter, Dr. Dipl.-Ing.

(57) **Zusammenfassung**

Eine Eingabeeinheit (1) zur wahlweisen Beschickung eines Analysengerätes mit flüssigen oder gasförmigen Proben-, Referenz- und Reinigungsmedien weist eine Probeneingabeöffnung (2) und einen in einer Eingabeeinheit (1) einseitig gelagerten Zuführteil auf. Im Zuführteil (1) ist ein Anschluß (5) für das zuzuführende Reinigungsmedium angeordnet, wobei die Probeneingabeöffnung (2) einen elastischen Einfüllmund (3) aufweist, welcher mit einem in den Einfüllmund (3) einführbaren, am Zuführteil befestigten Waschkörper (6) zusammenwirkt. Der Zuführteil ist als eine um eine feststehende Achse verschwenkbare Klappe (4) ausgeführt. Der Anschluß (5) für das Reinigungsmedium mündet im Waschkörper (6) und dient auch zur Zufuhr von flüssigen und gasförmigen Referenzmedien. Der Waschlörper (6) ist auf einem elastisch in der Klappe (4) befestigten Träger angeordnet und schmiegt sich bei geschlossener Klappe (4) an den elastischen Einfüllmund (3) an.

## Beschreibung

Die Erfindung bezieht sich auf eine Eingabeeinheit zur wahlweisen Beschickung eines Analysengerätes mit flüssigen oder gasförmigen Proben-, Referenz- und Reinigungsmedien, mit einer Probeneingabeöffnung und einem in der Eingabeeinheit einseitig gelagerten Zuführteil, mit einem Anschluß für das zuzuführende Reinigungsmedium, wobei die Probeneingabeöffnung einen elastischen Einfüllmund aufweist, welcher mit einem in den Einfüllmund einführbaren, am Zuführteil befestigten Waschkörper zusammenwirkt

Zur Durchführung von chemischen Analysen mit Analysengeräten, bei welchen die obenerwähnte Eingabeeinheit gedacht ist, wird die Probe über die Probeneingabeöffnung einer Meßzelle oder einem Meßzellenblock zugeführt, wo die jeweilige Probe mit Meßelementen in Kontakt gebracht wird.

Einrichtungen dieser Art sind z.B. zur Durchführung von Blutgasanalysen oder Elektrolytanalysen mit Hilfe von elektrochemischen oder optochemischen Sensoren bekannt. Beispielsweise können damit der pH-, der pCO₂- oder der pO₂-Wert, Elektrolytkonzentrationen oder Substrate, wie Glukose, Harnstoff, Kreatinin, Lektat u. dgl. in einer Blutprobe gemessen werden.

In der US-PS 3 874 850 wird ein Blutgasanalysator beschrieben, welcher eine durch eine Klappe verschließbare Eingabeöffnung für die Probe aufweist. Bei geschlossener Eingabeklappe wird eine Kappe mit Abstand über ein elastisches Endröhrchen der Eingabeöffnung gestülpt, sodaß eine durch die Eingabeöffnung ausströmende Waschlösung über die Kappe sowie eine daran anschließende, in der Klappe geführte Leitung in den Abfallbehälter abfließen kann. Die Referenzmedien werden nicht über die Probeneingabeöffnung zugeführt, sondern gelangen über andere Zuleitungen in den Meßzellenblock.

Um zu verhindern, daß die einzelnen Medien zwischen Eingabeöffnung und dem Meßzellenblock unterschiedliche Wege zurücklegen, wodurch es für Kalibrier- und Probenmedien zu unterschiedlichen Meßbedingungen kommt, wurde beispielsweise in der AT-PS 391 213 vorgeschlagen, einen feststehenden Zuführteil mit mehreren Eingabeelementen vorzusehen. Die eigentliche Probeneingabeöffnung ist an einer drehbaren Halterung angeordnet, sodaß jedes Eingabeelement nach einer rotatorischen Verlagerung der Halterung mit der Probeneingabeöffnung in dichtschließenden Kontakt bringbar ist. Auf diese Weise wird garantiert, daß für alle in den Meßkammerblock gelangenden Medien, ausgehend von der Probeneingabeöffnung, derselbe Weg zurückgelegt wird.

Eine ähnliche Lösung dieses Problems wird weiters in der AT-PS 389 589 vorgeschlagen. Die hier beschriebene Einrichtung zur wahlweisen Beschickung eines Analysengerätes weist eine feststehende Probeneingabeöffnung auf, wobei ein mit Eingabeelementen versehener Zuführteil vorgesehen ist, welcher translatorische oder rotatorische Bewegungen ausführt und jeweils ein Eingabeelement über der Probeneingabeöffnung positioniert. Mittels einer parallel zur zentrischen Achse der Probeneingabeöffnung wirkenden Hubvorrichtung wird dann das Eingabeelement in die Probeneingabeöffnung eingeführt.

Eine Eingabeeinheit der eingangs genannten Art wird beispielsweise in der AT-PS 372 788 beschrieben. Ein einseitig gelagerter Zuführteil weist einen Anschluß für die Zufuhr von Reinigungsmedien auf, wobei im Zuführteil ein Waschkörper befestigt ist, welcher nach einer Dreh- und Hubbewegung des Zuführteiles in einen elastischen Einfüllmund der Probeneingabeöffnung einführbar ist. Der Waschkörper begrenzt während des Reinigungsvorganges zusammen mit dem trichterförmigen Einfüllmund einen der Form des Einfüllmundes angepaßten Ringspalt, welcher über eine im Zuführteil angeordnete Zuleitung mit einer Reinigungslösung beaufschlagbar ist.

Neben bereits erprobter Vorteile der genannten Einrichtungen sind diese jedoch in mechanischer Hinsicht kompliziert aufgebaut, wobei insbesondere die kombinierten Hub- und Drehbewegungen große Anforderungen an die Steuerung der automatisierten Eingabevorgänge stellen.

Aufgabe der Erfindung ist es, unter Beibehaltung der Vorteile der bekannten Einrichtungen, insbesondere der Verwendung gleicher Probenwege, eine mechanisch einfachere Beschickungseinrichtung vorzuschlagen.

Diese Aufgabe wird erfindungsgemäß dadurch gelöst, daß der Zuführteil als eine um eine feststehende Achse verschwenkbare Klappe ausgeführt ist, daß der Anschluß für das Reinigungsmedium auch zur Zufuhr von flüssigen und gasförmigen Referenzmedien dient und im Waschkörper mündet, welcher auf einem elastisch in der Klappe befestigten Träger angeordnet ist, wobei sich der Waschkörper bei geschlossener Klappe an den elastischen Einfüllmund anschmiegt und bei geöffneter Klappe die Probeneingabeöffnung für die Probeneingabe freigibt. Vorteilhafterweise wird durch die erfindungsgemäße Einrichtung das Ziel gleicher Wege für alle Medien durch einen einfachen Klappenmechanismus gelöst, welcher ohne komplizierte Bewegungsabläufe in seiner Offenstellung den Weg für die Probeneingabe mittels Pipette, Kapillare oder Spritze freigibt und in seiner geschlossenen Stellung einen Anschluß zu den Referenz- und Reinigungsmedien herstellt. Durch die elastische Lagerung des Waschkörpers in der Klappe ist ein dichtes Anschmiegen des Waschkörpers an den oberen Rand des Einfüllmundes gewährleistet, wodurch auch Referenzgase verlustfrei zugeführt werden können.

Zur automatischen Erfassung der Position der Klappe (offen oder geschlossen) kann die Eingabeeinheit in einer Weiterbildung der Erfindung im Bereich der Klappe eine Anzeigeeinheit, vorzugsweise eine Lichtschranke aufweisen. Die Anzeigeeinheit kann mit der Steuereinheit des Analysengerätes verbunden sein, sodaß eine Fehlbedienung weitgehend ausgeschlossen ist.

Eine weitere Ausgestaltung der Erfindung sieht vor, daß die Klappe einen vorzugsweise magnetischen Verriegelungsmechanismus aufweist, welcher die Klappe in der geschlossenen Stellung an der Eingabeeinheit fixiert.

Schließlich sei als weiterer Vorteil der Erfindung angeführt, daß der Anschluß für die zuzuführenden Referenz- und Reinigungsmedien im Bereich des Klappenlagers von der Klappe auf die Eingabeeinheit übertritt und sich in mehrere Teilleitungen verzweigt, wobei auf der Eingabeeinheit Steuerelemente angeordnet sind, welche jeweils nur eine Teilleitung zur Zufuhr eines Referenz- oder Reinigungsmediums freigeben. Vorteilhafterweise verzweigt sich der Anschluß für die zuzuführenden Referenz- und Reinigungsmedien erst außerhalb des als Klappe ausgeführten Zuführteils in mehrere Teilleitungen, welche zu den einzelnen Medienbehältern führen, sodaß im Bereich des Klappenlagers nur ein einziger Anschluß Biegebeanspruchungen beim Öffnen und Schließen der Klappe ausgesetzt ist.

Die Erfindung wird im folgenden anhand eines Ausführungsbeispieles näher erläutert. Es zeigen:
Fig. 1 eine erfindungsgemäße Eingabeeinheit in einer Schnittdarstellung,
Fig. 2 die Eingabeeinheit nach Fig. 1 in einer Seitenansicht,
Fig. 3 die Lage der Eingabeeinheit nach Fig. 1 und Fig. 2 in einem Analysengerät sowie
Fig. 4 ein schematisches Übersichtsdiagramm der Zu- und Ableitung der Proben-, Referenz- oder Reinigungsmedien.

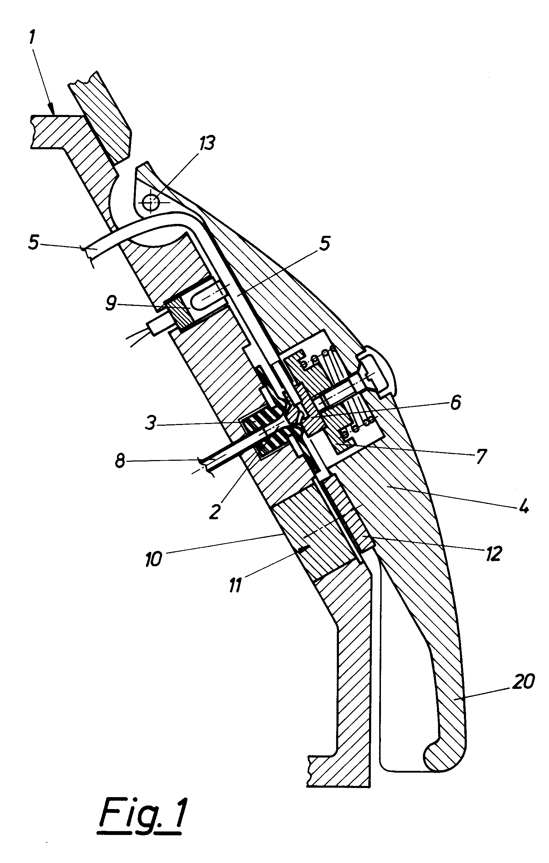

Fig. 1 stellt eine Eingabeeinheit 1 zur wahlweisen Beschickung eines Analysengerätes mit flüssigen und gasförmigen Medien dar, welche eine Probeneingabeöffnung 2 mit einem elastischen, trichterförmigen Einfüllmund 3 aufweist. Die Eingabeeinheit 1 weist weiters eine einseitig gelagerte, um eine feststehende Achse verschwenkbare Klappe 4 mit einem Anschluß 5 für die zuzuführenden Referenz- und Reinigungsmedien auf, welcher Anschluß 5 bei geschlossener Klappe 4 mit der Probeneingabeöffnung 2 bzw. dem Einfüllmund 3 zusammenwirkt. Der Anschluß 5 für die zuzuführenden Medien mündet in einen Waschkörper 6, welcher auf einem elastisch in der Klappe 4 befestigten Träger 7 angeordnet ist. Bei geschlossener Klappe 4 schmiegt sich der Waschkörper 6 an die Probeneingabeöffnung 2 bzw. den Einfüllmund 3 an, sodaß Reinigungs- sowie Referenzmedien über den Anschluß 5 in den Einfüllmund 3 und weiter in die Vorsaugstrecke 8 gesaugt werden können. Bei offener Klappenstellung ist die Probeneingabeöffnung 2 für die Eingabe von Proben mittels Spritze oder Pipette frei zugängig.

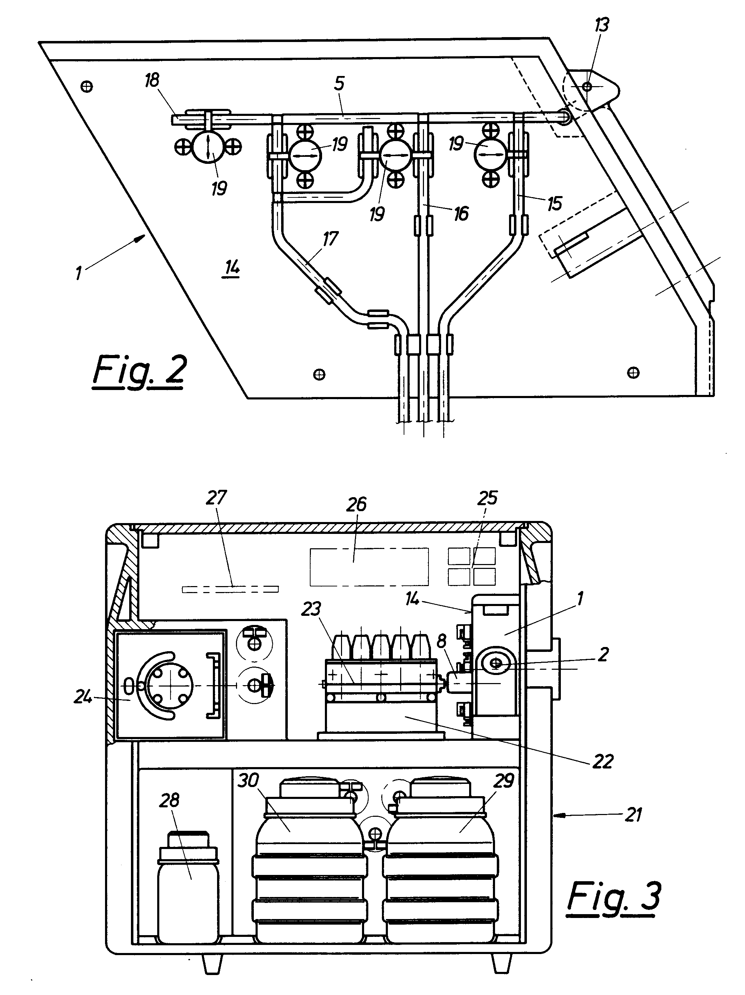

Die Eingabeeinheit 1 weist im Bereich der Klappe 4 eine Anzeigeeinheit 9 in Form einer Lichtschranke, eines Hallsensors oder Ultraschallgebers od. dgl. auf, welche die Stellung der Klappe 4 erfaßt und diese an eine Steuereinheit des Analysengerätes (Fig. 3) weiterleitet. Weiters ist in der Klappe 4 ein Verriegelungsmechanismus 10, beispielsweise ein Topfmagnet 11 in der Eingabeeinheit und eine ferromagnetische Platte 12 in der Klappe 4, vorgesehen, wodurch die Klappe in der geschlossenen Stellung fixiert wird und mit Hilfe des Griffstückes 20 entriegelt werden kann.

Der Anschluß 5 für die Referenz- und Reinigungsmedien tritt im Bereich des Klappenlagers 13 von der Klappe 4 auf die Eingabeeinheit 1 über und verzweigt sich - wie aus Fig. 2 ersichtlich - an deren Seitenfläche 14 in mehrere Teilleitungen 15, 16, 17 und 18, welche über Steuerelemente 19, z.B. Magnetventile, geöffnet und geschlossen werden können.

Die Lage der erfindungsgemäßen Eingabeeinheit 1 in einem Analysengerät 21 geht aus Fig. 3 hervor. Das Analysengerät ist hier in einer Frontansicht ohne Deckel, bei abgenommener Klappe dargestellt. An die Vorsaugstrecke 8 der Eingabeeinheit 1 ist direkt ein Meßkammerblock 22 angeschlossen, welcher parallel zu seiner Meßkapilare 23 verschoben, von der Eingabeeinheit 1 abgekoppelt und aus dem Analysengerät 21 entnommen werden kann. Mit 24 ist eine Schlauchpumpe zur Beförderung der Medien, mit 25 ein Tastenfeld zur Bedienung des Analysengerätes sowie mit 26 bzw. 27 beispielsweise ein LCD-Display bzw. ein Printermodul zur Ausgabe der Analysenergebnisse bezeichnet. Im Analysengerät 21 sind auswechselbare Behälter 28, 29 für Referenz- und Reinigungsmedien, sowie ein Abfallbehälter 30 vorgesehen.

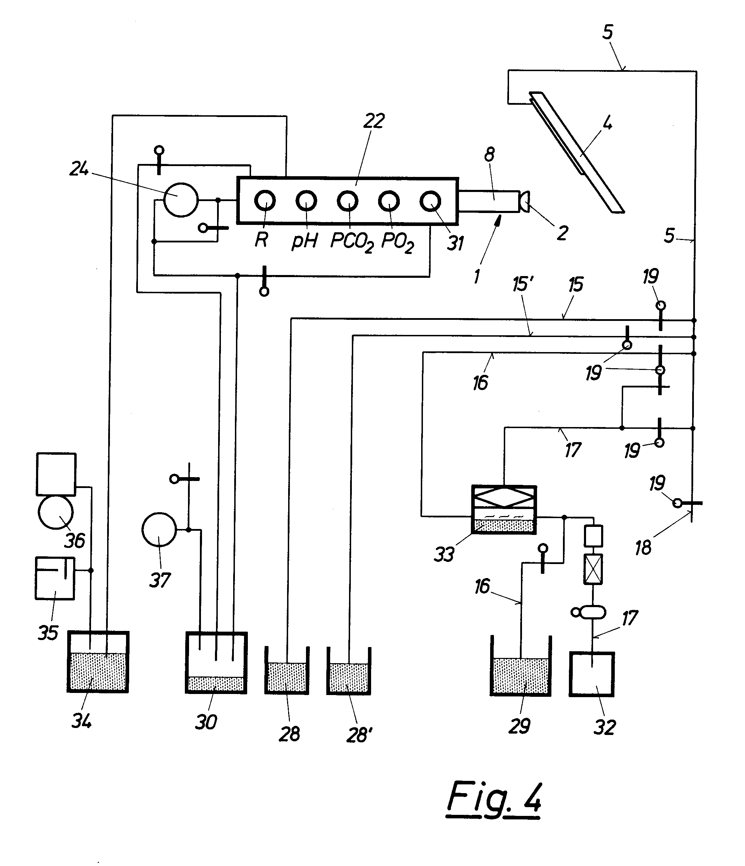

In Fig. 4 ist schematisch ein Meßkammerblock 22 mit Sensoren, z.B. mit pH-, PCO₂-, pO₂-Elektroden, mit einer Referenzelektrode R sowie einem Meßkammerventil 31 dargestellt. Über die Probeneingabeöffnung 2 der Eingabeeinheit 1 können mittels der Klappe 4 und deren Anschluß 5 je nach Stellung der Steuerorgane 19 unterschiedliche Referenz- und Reinigungsmedien in den Analysenweg eingebracht werden. So werden über die Leitungen 15 bzw. 15' aus den Behältern 28 bzw. 28' Referenzflüssigkeiten (Puffer 1 und Puffer 2) und aus dem Gasbehälter 32 ein Präzisionsgas (Teilleitung 17) zugeführt. Das gasförmige Referenzmedium wird in einem von der Reinigungsflüssigkeit durchströmten Gasbefeuchter 33 angefeuchtet. Die Waschlösung gelangt aus dem Behälter 29 über die Leitung 16 und die Klappe 4 in die Probeneingabeöffnung 2. Über die Teilleitung 18 kann weiters Luft angesaugt werden. Im Behälter 34, welchem eine KCl-Falle 35 und eine Druckpumpe 36 vorgeschaltet sind, befindet sich ein Referenzelektrolyt für die Referenzelektrode R. Der Abfallbehälter 30 steht mit einer Vakuumpumpe 37 in Verbindung.

## Patentansprüche

1. Eingabeeinheit zur wahlweisen Beschickung eines Analysengerätes mit flüssigen oder gasförmigen Proben-, Referenz- und Reinigungsmedien, mit einer Probeneingabeöffnung (2) und einem in der Eingabeeinheit (1) einseitig gelagerten Zuführteil, mit einem Anschluß (5) für das zuzuführende Reinigungsmedium, wobei die Probeneingabeöffnung (2) einen elastischen Einfüllmund (3) aufweist, welcher mit einem in den Einfüllmund (3) einführbaren, am Zuführteil befestigten Waschkörper (6) zusammenwirkt, **dadurch gekennzeichnet**, daß der Zuführteil als eine um eine feststehende Achse verschwenkbare Klappe (4) ausgeführt ist, daß der Anschluß (5) für das Reinigungsmedium auch zur Zufuhr von flüssigen und gasförmigen Referenzmedien dient und im Waschkörper (6) mündet, welcher auf einem elastisch in der Klappe (4) befestigten Träger (7) angeordnet ist, wobei sich der Waschkörper (6) bei geschlossener Klappe (4) an den elastischen Einfüllmund (3) anschmiegt und bei geöffneter Klappe (4) die Probeneingabeöffnung (2) für die Probeneingabe freigibt.

2. Eingabeeinheit nach Anspruch 1, **dadurch gekennzeichnet,** daß im Bereich der Klappe (4) eine deren Position erfassende Anzeigeeinheit (9), vorzugsweise eine Lichtschranke, vorgesehen ist.

3. Eingabeeinheit nach Anspruch 1 oder 2, **dadurch gekennzeichnet,** daß die Klappe (4) einen vorzugsweise magnetischen Verriegelungsmechanismus (10) aufweist, welcher die Klappe in der geschlossenen Stellung an der Eingabeeinheit (1) fixiert.

4. Eingabeeinheit nach einem der Ansprüche 1 bis 3, **dadurch gekennzeichnet,** daß der Anschluß (5) für die zuzuführenden Referenz- und Reinigungsmedien im Bereich des Klappenlagers (13) von der Klappe (4) auf die Eingabeeinheit (1) übertritt und sich in mehrere Teilleitungen (15 bis 18) verzweigt, wobei auf der Eingabeeinneit (1) Steuerelemente (19) angeordnet sind, welche jeweils nur eine Teilleitung (15 bis 18) zur Zufuhr eines Referenz- oder Reinigungsmediums freigeben.
